# EUROPEAN PATENT APPLICATION

(11) **EP 3 375 362 A1**
(43) Date of publication of application: **19.09.2018**
(21) Application number: 17161257.5
(22) Date of filing: 16.03.2017
(51) Int. Cl.: A61B 5/024, A61B 5/05, A61B 5/11, A61B 5/113, A61B 5/117, G07C 9/00

(54) **CARDIO BIOMETRIC APPARATUS FOR SMART DOORS OR GATES**

(71) Applicant: Gunnebo Entrance Control Limited, Uckfield, East Sussex TN22 1QQ (GB)
(72) Inventor: PORT, Iain David, West Sussex, Sussex BN43 5LH (GB)
(74) Representative: Burchielli, Riccardo

(57) **Abstract**

A cardio-biometric apparatus (10) for smart doors comprising:
- a cardio device (11) capable to transmits wireless signal and receive a reflected signal from a user and extract a heart rate signature from said reflected signal;
- an authenticating means (12), adapted to receive said heart rate signature from said cardio device (11), made-up of at least;
- a coder (13) capable to transform said heart rate signature in to a user's authenticating data;
- a database (14) where all personal data of users, linked to said authenticating data, are pre-stored;
- an inter-communicating device (15) that communicates with said coder (13) and said database (14) to assess the authenticity of the user;
- an electronic lock device (16) capable of receiving command from said authenticating means (12) to lock or unlock a smart door according to said command it receives from said authenticating means (12).

## Description

### FIELD OF THE INVENTION

The present invention relates to biometric access control systems for smart doors.

Biometric access control is one of access method that is making companies, homes and other institutions a whole lot safer and biometric access control is also making our lives that little bit easier.

Not only are these systems highly effective for keeping people and companies safe but they are also cost effective. Biometric access control systems allow every employee to have their own access details that will allow their entrance and exit of the building to be tracked carefully. Not only does this minimize the possibility of crime happening but it also helps with determining the hours that an employee has worked.

In particular, this invention relates to the using of heart rate and its activities as a user's authenticating password. This form of biometric is unique in its nature. Since heart rate is based on the size and shape of the heart and the orientation of the valves and the user's physiology. The heart can beat faster or slower but electrically the beats look the same.

So, whether it beats faster or slower, it doesn't really matter. It's really about the shape of the waves, and what that signal looks like when it comes off the heart.

Though there are other spectrums of biometrics. So if you think about the uniqueness of different biometrics, retina and iris are the most unique but are very expensive.

Fingerprints are probably next but they can be temporary altered or destroyed by use of abrasive materials or when in contact with acids or base and then cardiogram (CG). CG metrics may not be as developed as they are with fingerprints, although it's approaching fingerprint uniqueness, but will exceed those metrics in the next few years. However, CG metrics are already far above things like voice recognition.

Unfortunately, typical technologies for tracking vital signals such as heartbeat and breathing require body contact, and most of them are intrusive. Some heart rate monitoring technologies are stressful or inconvenient since they require their users to wear a chest strap, or place a pulse oximeter on their finger. The more comfortable technologies such as wristbands have lower accuracy for heart rate monitoring. Additionally, there is a section of the population for whom wearable sensors are undesirable.

For example, the elderly typically feel encumbered or ashamed by wearable devices, and those with dementia may forget to wear them. Children may remove them and lose them, and infants may develop skin irritation from wearable sensors.

Therefore, the present invention falls within the field of wireless CG metric, thus using the heartbeat or heart rate to authenticate access at smart gates.

### BACKGROUND OF THE INVENTION

To date, the technology of CG metrics is used as security measure to allow access of staffs at smart doors. In this cited patent (US 2016/0048672 A1) by California Institute of Technology, the system and methods for identifying and(or) authenticating individuals utilizing microwave sending modules. In the embodiment, a microwave sensor module for authenticating a person using data related the person's heartbeat includes at least one receiver configured to receive a reflected microwave signal from a person that contains information related to the person's heartbeat, a processor, a memory containing an authenticating application, wherein the authentication application configures the processor to extract heartbeat data from the reflected signal related to displacement as a result of the person's heartbeat, compare the extracted heartbeat data against at least one template profile, and authenticate the person based upon the comparison of the extracted heartbeat data and the at least one template profile.

The proposed methods are more appropriate for controlled settings but unsuitable for smart doors.

The underlying problems related to this present invention are:
- They fail in the presence of multiple users or extraneous motion. They typically require the user to be still or face the device.
- Another problem is that they are accurate only when they are within close proximity to the user's chest.

Early work presented a proof of concept that the wireless signal is affected by movements of the chest. In present inventions, the person has to be stationary and the sensor has to be only few centimeters away from the apex of the heart. The results are qualitative with no evaluation of accuracy.
- Past proposals use one of the following techniques: Doppler radar, Wi-Fi, mobile signals, Bluetooth, or ultra-wideband radar. The key challenge in using wireless signals to extract heartbeats signs is that any motion in the surrounding affects the signal. Since heartbeats are minute movements, they can be easily overshadowed by interference from any other source of movement in the surrounding.
- Furthermore, the presence of multiple users - even if none of them moves - prevents these systems from operating correctly since the wireless signal will be affected by the combination of their heartbeats, making it hard to disentangle the heartbeat of each individual.

Past proposals deal with this problem by ensuring that there is only one source of motion in the surrounding: namely, the heartbeat of the monitored individual. Hence, the set-up has one person, who typically is at a still position in close proximity to the device.

### SUMMARY OF THE INVENTION

The principal purpose of the present invention is to provide a cardio biometric apparatus for smart doors which uses wireless signals to offer solutions to the drawbacks of the present invention described above.

Another purpose is to provide a cardio biometric apparatus for smart doors that can read heart rate from within a radius of 8 meters and authenticate personnel approaching a smart door or gate.

A further purpose is to provide a cardio biometric apparatus for smart doors that can process the presence of multiple users at a smart gate.

A Still a further purpose is to provide a cardio biometric apparatus for smart doors that uses wireless signals and not affected by any motion in the surrounding.

A much further purpose is to allow an employee to have his own access details that will allow him entrance and exit of the building to be tracked carefully. Not only does this minimize the possibility of crime happening but it also helps with determining the hours that an employee has worked.

Further characteristics and advantages of the present invention will be apparent from the following the detailed description of preferred but not exclusive embodiments of the cardio biometric apparatus for smart doors according to the claim 1 attached.

### BRIEF DESCRIPTION OF DRAWINGS

Detailed characteristics of the cardio biometric apparatus for smart doors according to the present invention are stated in the corresponding dependent claims.

Ulterior characteristics and advantages of the present invention majorly results from the description of one form of preferred execution but not exclusive of a cardio biometric apparatus for smart doors according to the present invention, illustrated by a non-limitative example in the accompanying drawings, in which:

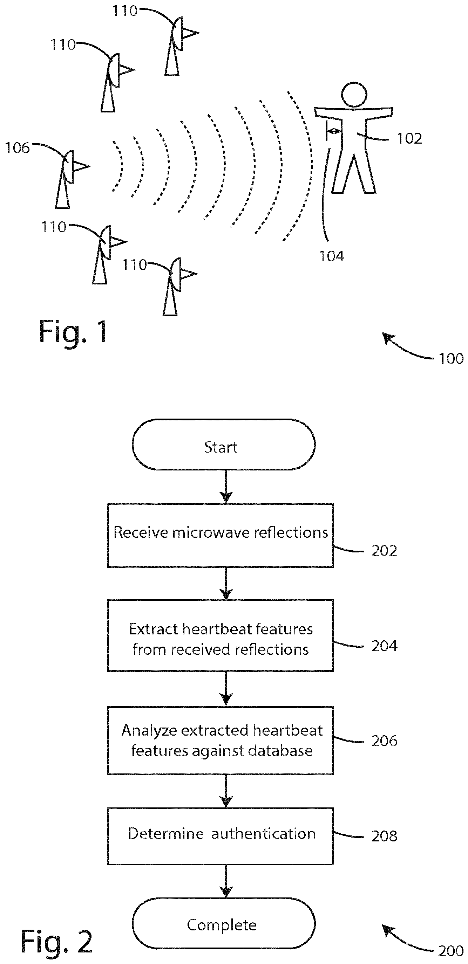

- Figure 1 illustrates design of the cited prior art.
- Figure 2 illustrates flow chart of cited prior art.
- Figure 3a illustrates design of apparatus of present invention, according to a first and a second embodiment.
- Figure 3b illustrates design of apparatus of present invention, according to a third embodiment.

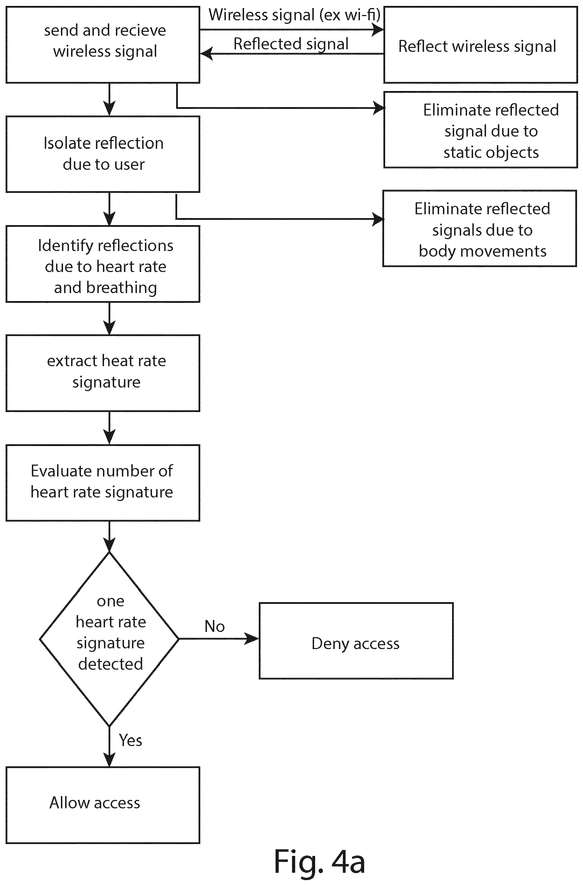

- Figure 4a illustrates flow chart of present invention, according to a first and a second embodiment.

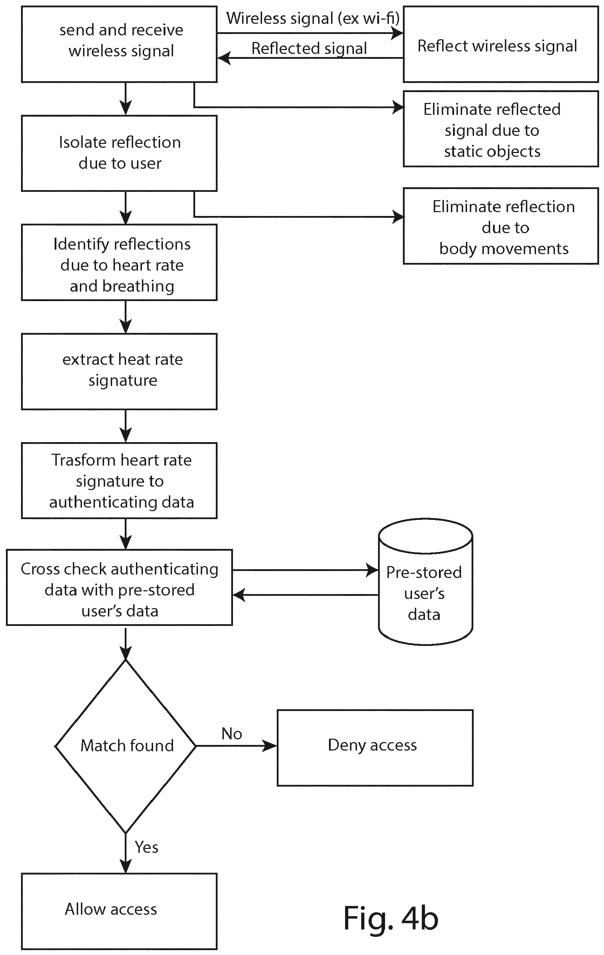

- Figure 4b illustrates flow chart of present invention, according to a third embodiment.
- Figure 5 illustrates phase variation due to vital signs.
- Figure 6 illustrates output of fourier transformation for heart rate.
- Figure 7 illustrates heart rate accuracy versus distance.

It is noted that anything found to be already known during the patenting process is understood not to be claimed and to be the subject of a disclaimer.

With reference to the figures, the reference numeral 10 generally designates a cardio biometric apparatus for smart doors comprising
- a cardio device 11 capable to transmit wireless signal to a controlled area and measures the time it takes its signal to travel to a human body and reflect back to its antenna and retrieving a heart rate signature from the signal received (by "heart rate signature", we intend at least a period of the shape of the ECG spikes),
- an authenticating means 12 adapted to receive heart rate signature signal from the cardio device 11,
- an electronic lock system 16 capable of receiving command from the authenticating means 12 to lock or unlock the smart door according to the information it receives from the authenticating means 12.

Knowing that wireless signals travel at the speed of light, the reflection time can be used to compute the distance from the cardio device 11 to the human body. This device can be integrated with the smart door but in this present invention, the cardio device 11 is preferably placed at a close proximity and focuses on the passage area of the smart door.

This distance varies slightly and periodically as the user inhales and exhales and his heart beats. The cardio device 11 captures these minute changes in distance and uses them to extract the user's vital signs.

However, the environment may have a some reflectors, such as wall or other static objects, as well as multiple users whose bodies all reflect the wireless signal. To address these issues, cardio biometric apparatus 10 for smart doors operation comprises of at least these steps:
1. Cardio device 11 send, receives and isolate reflections of signals from different users and eliminate reflections off static objects.
2. For each user, identify the signal variations that are due to breathing and heartbeats, and separate them from variations due to body or limb motion.
3. Analyze signal variations to extract heart rate signature.
4. Send the extract to the authenticating means 12 which is apt to count the number of heart rate signature extracts from the cardio device 11 into the user's authenticating data (heart rate signature graph or the personal identification code or both).
5. The authenticating means 12 then sends an access signal or deny signal to the electronic lock device 16 according to the number of heart rate signature detected. Smart door opens or remain locked according to the command it receives from the authenticating means 12 respectively.

### Step 1: Isolating Reflections from Users and Eliminate Reflections off static objects.

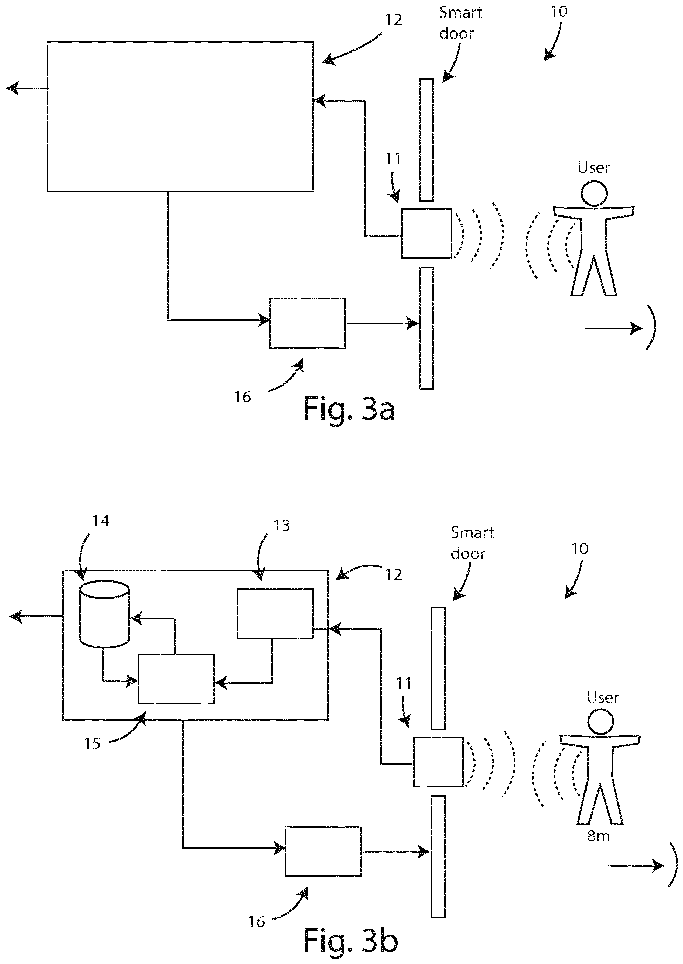

The cardio device 11 operates in this way, in Fig. 3 but there can be other variants (for example the device 11 integrated with the smart door), the cardio device 11 is located within the smart door 10 in close proximity and focuses on the passage area of the smart door. When the cardio device 11, preferably having a sensing device that can sense an approaching user within a range of 8 meters, transmits a wireless signal, this signal is reflected off the user approaching the door, back to a receiver of the cardio device 11. To isolate signals reflected off different objects, it computes a Fast Fourier transform (FFT) and the receiver can separate these signals. Separating the reflections from different objects into buckets based on their reflection times. Since wireless signals travel at the speed of light, signals reflected off objects at different distances would fall into different buckets. it uses this method to isolate the reflected signals arriving from different distances in the environment into different buckets, before it proceeds to analyze the signals in each of these buckets to extract the vital signs.

Reflections from two objects that are separated by at least 8 cm would fall into different buckets. Hence, two users that are few feet apart would naturally fall into different buckets.

Using FFT as a filter also allows us to isolate some of the limb motion from chest movements due to breathing and heartbeats. For example, the signal reflected off the user's feet will be in a different bucket from that reflected off the user's chest. Thus, having the user move his feet (in place) does not interfere with the cardio device's 11 ability to extract the breathing and heart rate. After bucketing the reflections based on the reflector's distance, the cardio device 11 eliminates reflections off static objects like walls and glass or metal surroundings. Specifically, since static objects don't move, their reflections don't change over time, and hence can be eliminated by subtracting consecutive time measurements. At the end of this step, the cardio device 11 would have eliminated all signal reflections from static objects(e.g, walls and metal or glass surroundings),and is left with reflections off moving objects separated into buckets.

### Step 2: Identifying Reflections Involving Breathing and Heart Rate.

After the cardio device 11 isolates reflections from different moving users into separate buckets, it proceeds by analyzing each of these buckets to identify breathing and heart rate. For example, in Fig. 3, to identify whether the user in a specific bucket is quasi-static and his motion is dominated by his vital signs, or whether he is walking around or moving a limb. To do that, cardio device 11 zooms in on the signal reflection which it isolated in the that corresponding bucket. This wireless reflection is a wave; the phase of the wave is related to the distance traveled by the signal as follows: ϕ(t) = 2π d(t) λ, where λ is the wavelength of the transmitted signal, and d(t) is the traveled distance from the cardio device 11 to the reflector and back to the cardio device 11. The above equation shows that one can identify variations in d(t) due to inhaling, exhaling, and heartbeats, by measuring the resulting variations in the phase of the reflected signal.

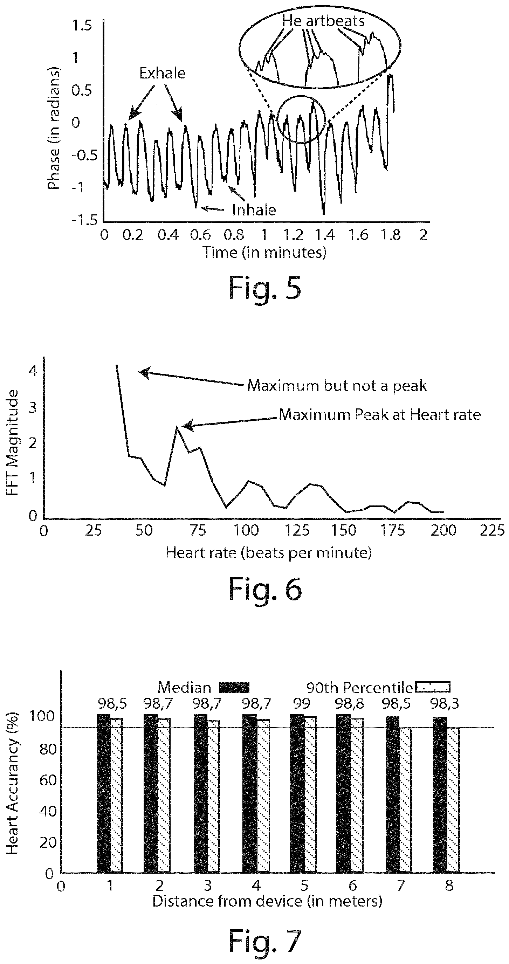

To illustrate how the phase varies with vital signs, we consider an example, where a user stands, facing the cardio device 11. When the person inhales, his chest expands and gets closer to the cardio device 11; and when he exhales, his chest contracts and gets further away from the cardio device 11. Because the phase and the distance to a reflector are linearly related, the cardio device 11 can track a person's breathing. Fig.5 shows the phase of the captured reflection as a function of time.

Specifically, a peak in the phase corresponds to an exhale (highest distance from the cardio device 11), and a valley in the phase corresponds to an inhale (smallest distance from the cardio device 11). According to the above equation, sub-centimeter variations in the chest distance due to breathing cause sub-radian variations in the phase, which is what is observed. Similarly, a user's heartbeats cause minute movements of different parts of his body. Specifically, the physiological phenomenon that allows the cardio device 11 to extract heart rate from signal reflections is ballistocardiography (BCG). BCG refers to movements of the body synchronous with the heart beat due to ventricular pump activity. Past work has documented BCG jitters from the head, torso, buttock, etc. Periodic jitters cause periodic variations in the wireless signal allowing the cardio device 11 to capture the heart rate. These movements translate to smaller fluctuations on top of the breathing motion in the wireless reflection as we can see from local peaks in Fig. 5. Note that the periodicity of breathing and heartbeats is independent of the user's orientation. For example, if the user has his back to the device, the valleys become peaks and vice versa, but the same periodicity persists. Still, an important question to answer is: what happens when a person moves around or moves a limb, and how can the cardio device 11 distinguish such motions from breathing and heart beats?

To help answer this question, a scenario where the user waves his hand before the one minute mark resulting in a periodic phase variations of the signal. To deal with such scenarios, the cardio device 11 exploits that motion due to vital signs is periodic, while body or limb motion is non periodic. It uses this property to identify intervals of time where a user's whole body moves or where the user performs large limb movements and discards them so that they do not create errors in estimating vital signs. To achieve this, the cardio device 11 operates on time windows (30 seconds in the implementation). For each window, the cardio device 11 measures the periodicity of the signal. If the periodicity is above a threshold, it determines that the dominant motion is breathing and heart rate; otherwise, it discards the window. A typical approach to measure a signal's periodicity is evaluating the sharpness of the cardio device's Fourier transform (or FFT). Hence, an FFT is perform on each window, choose the FFT's peak frequency, and determine whether the peak's value is sufficiently higher than the average power in the remaining frequencies.

This metric allows to maintain intervals where a user does not perform large limb movements, including scenarios where the user checks his or her phone. This is because, while these movements are indeed a periodic, they do not mask the breathing or the heart rate since their power does not overwhelm the repetitive movements due to our vital signs.

Additionally, in some of these scenarios, the user's hands are stretched out and away from his chest as he makes movements. As a result, the major part of his motion falls into a separate bucket than the user's chest.

Naturally, because the human body is connected, hand movements would still result in muscle stretches and minor shoulder jitters that are close to the user's chest; however, because such movements are weak and non periodic, they are diluted at the out put of the FFT. In contrast, periodic movements due to vital signs are enforced in the FFT operation, which results in maintaining intervals of such quasi-static scenarios. The above steps allow to filter out extraneous motion and focus on time windows where the dominant motion for each user is the breathing and heart rate.

### Step 3: Extracting Heart Rate signature.

The heartbeat signal is periodic, and is modulated on top of the breathing signal, as shown in Fig. 6.

However, the breathing signal is orders of magnitude stronger than the heartbeat. This leads to a classical problem in FFT's, where a strong signal at a given frequency leaks into other frequencies (i.e., leaks into nearby bins at the output of the FFT) and could mask a weaker signal at a nearby frequency.

To mitigate this leakage, the frequency domain signal is filtered around [40-200] beats per minute; this allows us to filter out breathing, which is typically between 8 and 16 breaths per minute as well as high frequency noise (which is higher than 200 beats per minute), leaving alone the heart rate signature.

Meanwhile, having found out that the heart rate signature varies from person to person and since everyone has his or her own unique heart rate signature, which remains even during heart rate changes caused by excitement or exercise. The signature stays the same over time. The cardio device 11 sends this unique heart signature to the authenticating means 12 to be processed.

In this present invention, preferably (not limiting the scope of the invention), the wireless signal is a Wi-Fi signal.

In another embodiment the wireless signal used is an ultrasonic wave.

In another embodiment the wireless signal used is a thermal or infrared signal.

In another embodiment the wireless signal used is a microwave (radio) signal (ultra-wideband radar or Continues waves).

In another embodiment the wireless signal used is a Frequency Modulated Carrier Waves (FMCW).

For preference, in this present invention (not limiting the scope of the invention), the above can be applied to security doors at offices, banks, airports and other restricted buildings that allows only authorized officials access. For the sake of understanding, we take an example of the entrance and exit of a work place. A smart door capable of allowing entrance of at least one user using their heart rate signature as their personal Identification code. The building entrance for authorized users have at least one automatic door controlled by the cardio biometrics to allow access of users. In another embodiment the entrance may have two automatic doors in series. One directly in front the other. A first automatic door that is controlled by a motion sensor detecting a user within a certain radius and allowing access into a chamber defined by the two automatic doors and two adjacent walls parallel to each other and perpendicular to the two automatic doors. A second automatic door controlled by cardio biometrics. For simplicity we take a case of one automatic door that opens only by heart rate signature of an authorized user. Preferably one user at a time. The smart door 10, equipped with the cardio device 11 placed in a close proximity or integrated with the cardio device 11. Although the cardio device 11 is capable of reading heart rate signatures within an 8 meter radius, we will concentrate on a person in front of the smart door 10. The cardio device 11 sends a Wi-Fi signal in the direction of the user. This Wi-Fi signal is reflected by the user back to the receiver of the cardio device 11. The cardio device 11 extracts the heart rate signature of the user from the reflected Wi-Fi signal it received. The Wi-Fi signal reflected of static objects are deleted by the cardio device 11. Also non periodic Wi-Fi signal received are also filtered out. The cardio device keeps only the periodic Wi-Fi signal it receives and later filters out the breathing signal and noise signal leaving that of the heart rate signature alone. The cardio device 11 now sends this signature to the authenticating means 12 for processing.

In this first embodiment, described above, we are not interested in knowing the identity of a user but rather to allow access to only one user at a time. For example, controlled gates where one person has access but more than one enter. As this happens often. For example, at a controlled door for underground train stations where one can pass through controlled door by staying very close to a user with access to the extent that the system considers them as a single entity or a group of fraudsters can organize and with one pass, they can hold each other closely and the system of the controlled door again considers them as a single unit.

### Step 4: Authenticating the Number of Users at smart door.

Thus, in this case the authenticating means 12 is configured to allow access only and only if there is one user, to be specific if it detects or counts one heart rate signature. To do this the authenticating means 12 preferably has a counter (not shown in the figures) to count the number of heart rate signatures detected by the cardio device 11 which correspond to the number of users at the smart door or the authenticating means 12 might comprise of any device adapted to count the number of users by counting the number of heart rate signatures at the smart door.

### Step 5: Access or Denial command to the Electronic Lock Device 16.

If the authenticating means 12 detects more that one heart rate signature, it denies access but on the contrary if it detects only one heart rate signature it allows access of the user by sending a command to the electronic lock system 16 to open the door for the user and close after the user has passed.

In this particular embodiment the cardio device 11 is placed in close vicinity of the smart door and configured to access heart rate signature of only a user close to the smart door within a certain radius from the smart door. This application is also suitable for booths, by the word "booth" we mean an enclosed compartment made of at least two smart doors and compartment wall or walls to define a room where a user can enter. The first smart door that allows access into the booth is preferably controlled by a motion sensor and the second door that allows exit of a user from the booth is controlled by the cardio device 11.

In a second embodiment, authenticating means 12 preferably comprises in addition to the first embodiment described above a monitoring device(not shown in the figures attached) suitable to monitor the position or progress of the user through a smart door using the heart rate signature. This is to ensure that only one user goes through the smart door, for example in a booth with smart doors, where a first door opens to allow access of a user and closes after the user enters the booth. When there is a user in the booth, the cardio device 11 extracts the heart rate signature of the user and sends to the authenticating means 12 and gives command to the lock system 16 to hold closed the first door, even if there are other users infront of the first door. The authenticating means 12 then evaluates the number of users in the booth using the heart rate signature. If its more than one, the authenticating means 12 gives command to the lock system 16 to hold closed a second door and to open the said first door so that the excess users can go out leaving only one in the booth. If only one user is detected, the said second door opens whilst the said first door remains closed and said monitoring 19 is adatpted to track the progress of the user's position till the user is out through the said second door and then closes the second door before allowing another user in the booth.

In a third embodiment, with reference to the figures, the reference numeral 30 generally designates a cardio biometric apparatus for smart doors comprising;
- a cardio device 11 capable to transmit wireless signal to a controlled area and measures the time it takes its signal to travel to a human body and reflect back to its antenna and retrieving a heart rate signature from the signal received (by "heart rate signature", we intend at least a period of the shape of the ECG spikes),
- an authenticating means 12 adapted to receive heart rate signature signal from the cardio device 11, comprising at least
- a coder 13 capable to plot graphically the recorded heart rate signature or convert the heart rate signature into a personal identification code or both,
- a database 14 where all personal data of users linked to their heart rate signature graph or personal identification code related to their heart rate signature or both are pre-stored,
- an inter-communicating device 15 that communicates with the coder and the database to assess the authenticity of the user,
- an electronic lock system 16 capable of receiving command from the authenticating means 12 to lock or unlock the smart door according to the information it receives from the authenticating means 12.

Knowing that wireless signals travel at the speed of light, the reflection time can be used to compute the distance from the cardio device 11 to the human body. This device can be integrated with the smart door but in this present invention, the cardio device 11 is preferably placed at a close proximity and focuses on the passage area of the smart door.

This distance varies slightly and periodically as the user inhales and exhales and his heart beats. The cardio device 11 captures these minute changes in distance and uses them to extract the user's vital signs.

However, the environment may have a some reflectors, such as wall or other static objects, as well as multiple users whose bodies all reflect the wireless signal. To address these issues, cardio biometric apparatus 10 for smart doors operation comprises of at least these steps:
1. Cardio device 11 send, receives and isolate reflections of signals from different users and eliminate reflections off static objects.
2. For each user, identify the signal variations that are due to breathing and heartbeats, and separate them from variations due to body or limb motion.
3. Analyze signal variations to extract heart rate signature.
4. Send the extract to a coder 13 within the authenticating means 12 which transforms heart rate signature extracts from the cardio device 11 into the user's authenticating data (heart rate signature graph or the personal identification code or both).
5. The inter-communicating device 15 receives the user's authenticating data from the coder and crosscheck it with pre-stored data of users to find a match in the database 14.
6. The inter-communicating device 15 then sends an access signal or deny signal to the electronic lock device 16 according to the match result. Smart gate opens or remain locked according to the command it receives from the inter-communicating device 15 respectively.

For Step 1, Step 2 and Step 3, refer to the first 3 steps of the first embodiment.

### Step 4: Transformation of heart rate signature into authenticating data.

This signal is received by the coder 13 of the authenticating means 12, the coder 12 plots graphically the heart rate signature or convert this signature into user's personal identification code or both.

The coder 13 then sends this authenticating data to the inter-communicating system 15 of the authenticating means 12.

### Step 5: Crosschecking authenticating data with pre-stored data.

Since the shape of the ECG spikes or the personal identification code of all authorized users are pre-stored in a database 14 within the authenticating means 12.

It is now the job of the inter-communicating system 15 to crosscheck this data received from the coder 13 with the database 14. Preferably, in this example, it crosschecks the heart rate signature graph using traditional methods of search and tries to find a match.

In another embodiment it uses the personal identification code.

The heart rate signature pre-stored in the database 14 is linked with all the authorized user's personal information. When the inter-communicating system 15 crosschecks and find finds a match, it confirms the authenticity of the user.

### Step 6: Access or Denial command to the Electronic Lock Device 16.

The inter-communicating system 15 then sends a command to the electronic lock system 16 to open the smart door 10. It also registers the presence of the user and records the time of entrance and stores it in the a folder linked to the user's personal data in the database 14. This data can be accessed to control user's daily presence. If on the contrary, does not find any information in the database 14 related to the user, it sends a command to the electronic lock system 16 not to open the smart door 10 and in a more secured setting, it may be programmed to sends a warning to a security control room, prompting them of the presence of an unauthorized user.

### Test of Heart Rate Reading Accuracy.

A plot of the median and 90th percentile accuracy of heart rate as a function of distance from 1 to 8 meters in Fig.7. The figure shows that the median accuracy is 98.5% at 1 meter and drops to 98.3% at 8 meters from the cardio device 11. It also shows that the 90th percentile accuracy remains higher than 90%, even with the subject being 8 meters away from the cardio device 11.

The invention thus conceived is susceptible of numerous modifications and variations, all of which are within the scope of the appended claims; all the details may further be replaced with other technically equivalent elements.

In practice, materials used, as well as the contingent shapes and dimensions, may be any according to the requirements and the state of art.

Where the constructional characteristics and techniques mentioned in the following claims are followed by reference signs or numbers, such signs and reference numbers have been applied with the sole purpose of increasing the intelligibility of the claims and consequently, they do not constitute in any way limiting the interpretation of each element identified, purely by way of example, by such signs and reference numbers.

## Claims

1. A cardio-biometric apparatus (10) for smart doors comprising:
- a cardio device (11) capable to transmit a wireless signal to a controlled area and receive a reflected signal from said controlled area from at least a user and extract a heart rate signature from said reflected signal;
- an authenticating means (12), adapted to receive said heart rate signature from said cardio device (11);
- an electronic lock device (16) capable of receiving command from said authenticating means (12) to lock or unlock at least one smart door according to said command it receives from said authenticating means (12).

2. A cardio biometric apparatus (10) for smart doors according to claim 1 **characterized in that** said authenticating means (12), adapted to receive said heart rate signature from said cardio device (11), comprising at least:
- a coder (13) capable to transform said heart rate signature in to a user's authenticating data;
- a database (14) where all personal data of users, linked to said authenticating data, are pre-stored;
- an inter-communicating device (15) that communicates with said coder (13) and said database (14) to assess the authenticity of the user.

3. A cardio biometric apparatus (10) for smart doors according to claim 1 **characterized in that** said authenticating means (12) is configured to detect the number of heart rate signature and to deny access if it detects more than one heart rate signature.

4. A cardio biometric apparatus (10) for smart doors according to claim 1 **characterized in that** said authenticating means (12) is configured to monitor the progress of the heart rate signature of a user through said smart door.

5. A cardio biometric apparatus (10) for smart doors according to claim 1 **characterized in that** said wireless signal is a Wi-Fi signal or a micro wave or a frequency modulated carrier wave (FMCW) or an ultra-sonic wave or an infrared.

6. A cardio biometric apparatus (10) for smart doors according to claim 2 **characterized in that** the authenticating data created by said coder (13) is a heart rate signature graph.

7. A cardio biometric apparatus (10) for smart doors according to claim 2 **characterized in that** said authenticating data created by said coder (13) is a personal identification code.

8. A cardio biometric apparatus (10) for smart doors according to claim 1 **characterized in that** the entrance have two automatic doors, one in front the other, wherein a first automatic door is controlled by a motion sensor detecting a user within a certain radius and allowing access into a chamber defined by the two automatic doors and chamber walls and a second automatic door is controlled by said cardio biometric apparatus (10).

9. A cardio biometric apparatus (10) for smart doors according to one of the above claim 9 **characterized in that** said cardio device extracts one heart rate signature at a time and said chamber allows one user at a time.

10. A method of authenticating a user for access at a smart door, using a heart rate signature, the method comprising:
- sending and receiving reflections of wireless signals, from a user, by a cardio device;
- isolate said reflections of signals from said user and eliminate reflections of static objects;
- identify said reflection signal variations that are due to breathing and heartbeats, and separate them from variations due to body or limb motion;
- analyze said signal variations and extract said heart rate signature;
- then sends an access signal or deny signal to an electronic lock device (16) according to the match result;
- so that said smart door opens or remain locked, respectively, according to the command it receives from said authenticating means (15).

11. A method of authenticating a user for access at a smart door, using said heart rate signature, according to claim 10, **characterized in that** after extracting the heart rate signature it:
- send said heart rate signature to a coder (13) within an authenticating means (12);
- transform said heart rate signature extracted from the cardio device (11) into user's authenticating data;
- send said user's authenticating data from said coder (13) to an inter-communicating device (15);
- crosscheck said authenticating data with pre-stored data of users to find a match in a database (14).

12. A method of authenticating a user for access at a smart door, using said heart rate signature, according to claim 11, **characterized in that** said authenticating data is constituted by a heart rate signature graph.

13. A method of authenticating a user for access at a smart door, using said heart rate signature, according to claim 11, **characterized in that** said authenticating data is constituted by a personal identification code derived from heart rate signature.

14. A method of authenticating a user for access at a smart door, using said heart rate signature, according to claim 10, **characterized in that** the authenticating means (12) is configured to detect the number of heart rate signature and to deny access at said smart door if said heart rate signature are more than one.
